**Europäisches Patentamt**

**European Patent Office**

**Office européen des brevets**

(19)

(11) Veröffentlichungsnummer: **0 068 047**
**B1**

(12)

# EUROPÄISCHE PATENTSCHRIFT

(45) Veröffentlichungstag der Patentschrift:
23.07.86

(21) Anmeldenummer: 81110615.2

(22) Anmeldetag: 18.12.81

(51) Int. Cl.⁴: **A 61 L 15/04,** A 61 L 15/03,
A 61 L 17/00, A 61 K 37/62,
A 61 K 37/54, A 61 K 37/475,
A 61 K 37/02, A 61 K 35/16

(54) Angereichertes Plasmaderivat zur Unterstützung von Wundverschluss und Wundheilung.

(30) Priorität: 25.06.81 DE 3124962

(43) Veröffentlichungstag der Anmeldung:
05.01.83 Patentblatt 83/1

(45) Bekanntmachung des Hinweises auf die Patenterteilung:
23.07.86 Patentblatt 86/30

(84) Benannte Vertragsstaaten:
AT BE CH DE FR GB IT LI LU NL SE

(56) Entgegenhaltungen:
EP-A-0 059 265
FR-A-2 446 900

ARCHIV FÜR ORTHOPÄDISCHE UND UNFALL-
CHIRURGIE, Band 90, Nr. 1, 1977, Seiten 63-65,
Bergman-Verlag, Wiesbaden, DE. P. BOSCH et al.:
"Die Technik der Fibrinspongiosaplastik"

(73) Patentinhaber: Serapharm GmbH & Co. KG, Kaiser-
Wilhelm- Ring 36, D-4400 Münster (DE)

(72) Erfinder: Serapharm GmbH & Co. KG, Kaiser-
Wilhelm- Ring 36, D-4400 Münster (DE)

(74) Vertreter: Brehm, Hans- Peter, Dr. Dipl.- Chem.,
Patentanwälte Tischer, Kern & Brehm Albert-
Rosshaupter- Strasse 65, D-8000 München 70 (DE)

LIBER, STOCKHOLM 1986

## Beschreibung

Die Erfindung betrifft ein angereichertes Plasmaderivat in Form eines zur Unterstützung von Wundverschluß und Wundheilung bestimmten biochemischen Substrates, wobei die Anwendung auf Kollagen als Trägermaterial ausgenommen ist, und dessen Zusammensetzung einen Gehalt an Fibrinogen, Thrombin, Komponenten des Prothrombinkomplexes und Proteaseinhibitoren einschließt und im Hinblick auf eine optimierte Aktivierung des exogenen und/oder endogenen Gerinnungssystems und unter Berücksichtigung einer Vielzahl physiologischer und gegebenenfalls pathologischer Gesichtspunkte ausgewählt ist. Weiterhin betrifft die Erfindung ein Verfahren zur Herstellung eines solchen Plasmaderivates.

Ein angereichertes Plasmaderivat dieser Art ist aus der französischen Patentschrift 2 448 900 (entspricht DE-OS 30 02 934) bekannt. Das bekannte Plasmaderivat ist ein sogenannter "Gewebeklebstoff", der neben geringen Mengen Faktor XIII und einem Fibrinolyse-Inhibitor wie etwa Aprotinin aus mindestens 33 Gew.-% Fibrinogen besteht und zusätzlich in einem bestimmten Verhältnis Albumin enthält.

Zur Herstellung wird humanes, bei -20°C eingefrorenes Frischplasma auf +2°C erwärmt. Das dabei entstandene Kryopräzipitat wird durch Zentrifugieren abgetrennt, mit einer bestimmt zusammengesetzten Pufferlösung behandelt und nochmals zentrifugiert. Der abgetrennte Niederschlag wird in einer weiteren Pufferlösung gelöst, welche Humanalbumin, Glycin, Aprotinin und Heparin enthält. Die erhaltene Lösung wird auf eine Konzentration von 70 mg Protein pro ml verdünnt. Die Lösung wird sterilfiltriert, tiefgefroren und lyophilisiert. Zur Bereitstellung eines gebrauchsfertigen Gewebeklebstoff-Präparates wird das Lyophilisat mit Aqua ad iniectabilia rekonstruiert und als wässrige Lösung mit einer Fibrinogenkonzentration von mindestens 70 mg/ml eingesetzt. Vor der Applikation dieses bekannten Gewebeklebstoffes auf dem zu verbindenden Gewebe wird eine Mischung aus Thrombin und Calciumchlorid der wässrigen Proteinlösung zugefügt oder auf das Gewebe aufgebracht.

In der Praxis taut man die tiefgefrorene Fibrinogenlösung auf, versetzt mit Thrombin und Calciumchlorid, hält das Gemisch eine zeitlang, bis sich die einsetzende Polymerisationsreaktion durch eine Viskositätssteigerung bemerkbar macht und bringt dieses reagierende Gemisch auf den zu verbindenden Gewebeteilen auf. Der Aufwand zur Bereitung des einsatzfähigen Gewebeklebers und die geringe Lebensdauer des einsatzbereiten Präparates hat sich in vielen Fällen als hinderlich erwiesen. Zum anderen ist die Handhabung für den praktizierenden Arzt schwierig, weil er das kurzzeitige Intervall eines noch flüssigen Klebers nicht sicher erfassen kann.

Weiterhin ist aus der älteren - nicht vorveröffentlichten - Europäischen Patentpublikation 59 265 ein Material zum Abdichten und Heilen von Wunden bekannt, das aus einem Kollagenträger besteht, der einseitig oder allseitig mit einer Mischung beschichtet ist, die Fibrinogen, Thrombin oder eine Thrombin freisetzende Substanz, und gegebenenfalls weitere übliche Zusätze wie Calciumionen, Protease-Inhibitoren und infektionshemmende Arzneimittel enthält. Um eine Thrombin und Fibrinogen enthaltende Mischung auf einem Kollagenträger aufzubringen, werden diese Substanzen in einem organischen Lösungsmittel suspendiert, die Suspension auf einer oder beiden Oberflächen des Kollagenträgers aufgetragen, und daraufhin das Lösungsmittel verdampft. Alternativ können die Fibrinogenkomponente und die Thrombinkomponente in fester Form auf einen Kollagenträger aufgebracht werden, der vorher mit einem vorwiegend aus einem organischen Lösungsmittel bestehenden Medium oder mit einer geringen Menge Wasser befeuchtet worden ist. Die direkte und unmittelbare Anwendung eines trockenen Pulvergemisches, das Fibrinogen und Thrombin enthält - unabhängig von einem Kollagenträger - zur Unterstützung von Wundverschluß und Wundheilung läßt sich diesem älteren Vorschlag nicht entnehmen.

Die Aufgabe der vorliegenden Erfindung besteht darin, ein angereichertes Plasmaderivat der oben bezeichneten Art bereitszustellen, das praktisch unbeschränkt bei Raumtemperatur lagerfähig ist und das unmittelbar, d.h. ohne Änderung seines Aggregatzustandes und ohne die Zugabe anderer notwendiger Komponenten auf der Wunde bzw. dem Operationsgebiet aufgebracht werden kann.

Ausgehend von einem angereicherten Plasmaderivat in Form eines zur Unterstützung von Wundverschluß und Wundheilung bestimmten biochemischen Substrates, wobei die Anwendung auf Kollagen als Trägermaterial ausgenommen ist, und dessen Zusammensetzung einen Gehalt an Fibrinogen, Thrombin Komponenten des Prothrombinkomplexes und Proteaseinhibitoren einschließt und im Hinblick auf eine optimierte Aktivierung des exogenen und/oder endogenen Gerinnungssystems und unter Berücksichtigung einer Vielzahl physiologischer und gegebenenfalls pathologischer Gesichtspunkte ausgewählt ist, ist die erfindungsgemäße Lösung dieser Aufgabe dadurch gekennzeichnet, daß das Substrat gegebenenfalls zusätzlich Beimischungen von Blutplättchenextrakten, Antibiotika und dergleichen enthalten kann, alle Substratbestandteile ausnahmslos in pulverförmigem Zustand vorliegen, und das Substrat zur Applizierung in trockenem Zustand auf einer Wunde bzw. im Operationsgebiet konfektioniert ist. Das fertige Plasmaderivat bildet ein gut lagerfähiges Pulvergemisch aus ausschließlich trockenen, festen Komponenten, das unmittelbar in dieser festen Form auf der Wunde bzw. der Operationsstelle aufgebracht wird.

Insbesondere sieht die vorliegende Erfindung zur Lösung obiger Aufgabe ein angereichertes plasmaderivat zur Unterstützung von Wundverschluß und Wundheilung mit 60 bis 98 Gew.-% Fibrinogen und mit 0,05 bis 5 Gew.-% Fibrinolyse-Inhibitor vor, wobei

a) das Fibrinogen weitgehend frei von kälteunlöslichem Globulin ist;

b) der Gehalt an Thrombin und/oder Prothrombin 0,1 bis 15 Gew.-% ausmacht;

c) gegebenenfalls zusätzlich Phospholipide, Prostaglandine, Trockenhalte- und Stabilisierungsmittel, Antibiotika und/oder Blutgerinnungsfaktoren vorhanden sind;

d) alle Komponenten in biologisch aktiver, bei Temperaturen bis zu 56°C fester, pulverförmiger Form vorliegen; und

e) diese festen Komponenten miteinander vermischt sind.

Dieses erfindungsgemäße, angereicherte Plasmaderivat ist praktisch unbegrenzt lagerbar, in den Körperflüssigkeiten schnell löslich und kann daher ohne weitere Manipulationen unmittelbar und direkt zum Wundverschluß eingesetzt werden. Dank der Beimengung von Thrombin und/ oder Prothrombin tritt nach Anlösen und/oder Auflösen in Körperflüssigkeit eine schnelle Polymerisation ein, und es wird ein viskoses, gut haftendes Wundabdeckungsmaterial gebildet.

Die pulverförmige Konsistenz des Präparates erlaubt ein Zerstäuben, Versprühen oder Verschäumen mittels geeigneter Treibmittel, wodurch auch Gewebespalten oder Kavitäten abgedichtet werden können. Dank seines trockenen, festen, pulverförmigen Zustandes in Verbindung mit einer Kombination von Enzymen und Proenzymen im angereicherten Plasmaderivat ist dessen Lagerung besonders einfach.

Im Rahmen der vorliegenden Erfindung soll das aus Humanplasma isolierte Fibrinogen weitgehend frei von kälteunlöslichem Globulin sein. Die Anwesenheit von kälteunlöslichem Globulin beeinträchtigt die Fibrinbildung und hemmt die Wundheilung. Bezogen auf das Gesamtgewicht des pulverförmigen, angereicherten Plasmaderivates soll der Anteil an kälteunlöslichem Globulin weniger als 2 Gew.-%, vorzugsweise weniger als 0,4 Gew.-% und besonders bevorzugt weniger als 0,2 Gew.-% betragen. Je geringer der Anteil an kälteunlöslichem Globulin ist, desto schneller beginnt und verläuft die Fibrinpolymerisation.

Ein derartiges, an kälteunlöslichem Globulin weitgehend befreites Fibrinogen kann aus Humanplasma durch Fällung mit einem Glycin, β-Alanin und Athanol enthaltendem Lösungsmittelgemisch nach anschließender Dialyse und Lyophilisation des Fällungsproduktes erhalten werden. Vorzugsweise enthält das erfindungsgemäß angereicherte Plasmaderivat auf diesem Wege erhaltenes Fibrinogen. Derartiges Fibrinogen weist ein Molekulargewicht von 340 000 ± 5% auf, ist in der α-Kette leicht angedaut und liegt in mikrokristallinem Zustand vor. Bei Umgebungstemperatur löst sich derartiges Fibrinogen schnell in Körperflüssigkeit und beginnt unmittelbar darauf zu polymerisieren, beispielsweise in weniger als 2 min. Der in Lösung gerinnbare Anteil des Fibrinogens soll wenigstens 85 % betragen. Derartiges Fibrinogen wird auch als biologisch aktiv bezeichnet.

Das erfindungsgemäße, angereicherte Plasmaderivat enthält 0,05 bis 5 Gew.-% Fibrinolyse-Inhibitor. Vorzugsweise dienen als Fibrinolyse-Inhibitoren ein oder mehrere Antiplasmine. Beispielhafte Antiplasmine sind Aprothenin, $\alpha_1$-Antiplasmin und/oder Trypsin-Inhibitor. Der Zusatz derartiger Antiplasmine verhindert die Wiederauflösung des bereits gebildeten Fibringerinnsels.

Nach einem weiteren wichtigen Aspekt der vorliegenden Erfindung enthält das angereicherte Plasmaderivat das feste, biologisch aktive Fibrinogen unmittelbar vermischt mit festem, biologisch aktivem Prothrombin und/oder Thrombin. Der Anteil an Thrombin und/oder Prothrombin soll wenigstens 0,1 Gew.-% betragen. Bei geringeren Anteilen verläuft die Polymerisation des Fibrinogens verzögert und unvollständig. Thrombin und/oder Prothrombin-Anteile über 15 Gew.-% hinaus bringen keine zusätzliche Wirkung und sind daher im Hinblick auf die Kosten dieser Substanzen nicht ratsam. Vorzugsweise soll der Anteil an Thrombin und/oder Prothrombin 1 bis 10 Gew.-% betragen.

Bekanntlich dient biologisch aktives Thrombin als Start-Substanz für die Fibrinbildung und verkürzt die Reaktionszeit der Fibrinogen-Umwandlung. Biologisch aktives Thrombin im Sinne dieser Unterlagen liegt dann vor, wenn seine Aktivität unter den bekannten, standardisierten Bedingungen wenigstens 1000 internationale Einheit pro mg beträgt. Prothrombin dient als lagerstabile Thrombinreserve, aus dem sich das letztere beim Anfeuchten des Pulvers bildet. Das Prothrombin soll beim Einbringen in Körperflüssigkeit zu wenigstens 95 % in Thrombin umwandelbar sein.

Obwohl das erfindungsgemäße, angereicherte Plasmaderivat entweder Thrombin oder Prothrombin enthalten kann, wird die gemeinsame Anwesenheit von Thrombin und Prothrombin bevorzugt. Besonders bevorzugt sollen auf 1 Gew.-Teil Thrombin 0,1 bis 2 Gew.-Teile Prothrombin vorhanden sein. Die gemeinsame Anwesenheit gewährleistet eine hohe Aktivität auch nach langer Lagerdauer auch bei Einwirkung höherer Temperaturen, was beispielsweise für die Lagerung in den Tropen wertvoll sein kann. Ferner begünstigt die Kombination von Thrombin und Prothrombin die unmittelbare Umwandlung von Prothrombin zu Thrombin beim Anfeuchten des Pulvergemisches, so daß auch an der Wundstelle gebildete Gerinnungsfaktoren wie Faktor Xa zur Thrombinbildung beitragen können.

Das genannte System aus Fibrinogen, Thrombin und/oder Prothrombin und Fibrinolyse-

Inhibitor, alle in fester, pulverförmiger, biologisch aktiver Form bildet ein vollständiges System für den Wundverschluß, das in trockener Form unter sterilen Bedingungen beliebig lange lagerbar ist, jedoch nach Aufbringen auf der Wunde bzw. im Operationsgebiet durch An- und Auflösen in der Körperflüssigkeit gegebenenfalls unter Mitwirkung weiterer, in der Körperflüssigkeit enthaltener Faktoren biologisch aktiv wird. Vorzugsweise bildet dieses angereicherte Plasmaderivat ein mikrokristallines Pulvergemisch, bestehend aus 80 bis 94 Gew.-% Fibrinogen, 1 bis 10 Gew.-% Thrombin und/oder prothrombin und 0,01 bis 3 Gew.-% Fibrinolyse-Inhibitor, wobei der Gehalt an kälteunlöslichem Globulin weniger als 0,4 % des Gewichtes des gesamten Plasmaderivates ausmacht.

Obwohl die angegebenen Komponenten - Fibrinogen, Thrombin und/oder Prothrombin sowie Fibrinolyse-Inhibitor alle in fester, pulverförmiger, biologisch aktiver Form - einen vollständigen, direkt und unmittelbar, d.h. ohne weitere Manipulationen zur Anwendung geeigneten Gewebeklebstoff bilden, kann das Plasmaderivat zusätzlich weitere Bestandteile enthalten.

Vorzugsweise sind hierfür Phospholipide, Prostaglandine, Trockenhalte- und Stabilsierungsmittel, und/oder Blutgerinnungsfaktoren, alle in fester, pulverförmiger Form, vorgesehen. Der Anteil an Albumin ist nicht besonders kritisch und kann bis zu 35 %, vorzugsweise bis zu 15 % des Gewichtes des gesamten, angereicherten Plasmaderivates ausmachen. Gut geeignet als Trockenhalte- und Stabilisierungsmittel sind beispielsweise Albumin oder Globulin, das letztere etwa in Form des handelsüblich zugänglichen Gemisches aus $\alpha$, $\beta$ und $\gamma$-Globulin, oder ein Gemisch aus Albumin und Globulin, wobei Albumin bevorzugt eingesetzt wird. Die Anwesenheit von Prostaglandinen fördert die Aktivierung des Kapillarbettes im Wundgebiet, sowie die Aktivierung der im Blutstrom befindlichen Plättchen. Die Blutgerinnungsfaktoren, beispielsweise Faktor XIII, Blutplättchen-Extrakte und andere, zur Blutgerinnung notwendige Faktoren wie etwa Leukotriene, plättchenaktivierender Faktor, unterstützen und verstärken die Wirkung der in der Körperflüssigkeit vorhandenen Faktoren im Sinne einer akzelerierten Haemostase und einer Optimierung des Wundverschlusses. Als Phospholipid dient vorzugsweise ein aus humanem Vollblut gewonnener Thrombozytenextrakt. Weitere geeignete Phospholipide sind etwa Extrakte aus Gehirnsubstanz. Im Hinblick auf ihre hohe spezifische Wirksamkeit macht die Summe der Anteile an Prostaglandinen, Phospholipiden und Gerinnungsfaktoren zumeist nicht mehr als 1,2 Gew.-%, vorzugsweise nicht mehr als 0,85 Gew.-% des fertigen, angereicherten Plasmaderivates aus.

Weiterhin kann das erfindungsgemäße, angereicherte Plasmaderivat Antibiotika und andere, zur Bekämpfung bestimmter pathologischer Zustände wirksame Zusätze enthalten; hierzu gehören beispielsweise Penicillin, Antihisstaminika, Vasopressine und Gerinnungsfaktoren VIII oder IX zur haemophilen Wundversorgung.

Die genannten Bestandteile des erfindungsgemäßen angereicherten Plasmaderivates zur Unterstützung von Wundverschluß und Wundheilung sind als handelsübliche Präparate zugänglich oder können nach bekannten Verfahren hergestellt werden. Ohne die Erfindung darauf zu beschränken, ist nachstehend je ein Verfahren zur Gewinnung der wesentlichen Komponenten für das erfindungsgemäße, angereicherte Plasmaderivat angegeben.

### Gewinnung des Human-Fibrinogens:

Human-Plasma wird auf 4°C gekühlt und unter Rühren mit ß-Alanin (2 molare Lösung in Äthanol) versetzt, bis unter weiterer Äthanol-Zugabe das Rohfibrinogen ausfällt. Dieses Rohfibrinogen wird abzentrifugiert, in 0,01 M Tris-Puffer (pH-Wert 7,4) gelöst und durch Zusatz von 2 M Glycin erneut gefällt. Das abgetrennte Sediment wird in 0,9 %-iger wässriger NaCl-Lösung gelöst, gegen das gleiche Lösungsmittel dialysiert, entsalzt, und anschließend lyophilisiert.

Das Molekulargewicht des danach erhaltenen Präparates liegt im Durchschnitt bei 340 000. Die $\alpha$, $\beta$ und $\gamma$-Ketten sind nach Reduktion mit Mercaptoäthanol in der Gelelektrophorese gut nachweisbar. Der molare Extinktionskoeffizient $E^{1cm}_{280}$ liegt bei 16,0. Der Extinktionszuwachs bei alkalischer Hydrolyse beträgt 12 %. Der Anteil an kälteunlöslichem Globulin beträgt weniger als 0,2 Gew.-%, was durch Immunelektrophorese oder Radialimmundiffusion bestimmt werden kann.

Weiterhin sind handelsübliche Fibrinogenpräparate brauchbar, bei deren Gewinnung auf eine weitgehende Abtrennung des kälteunlöslichem Globulins geachtet wurde. Ein brauchbares, festes, pulverförmiges Human-Fibrinogen ist beispielsweise das von der Firma Behring-Werke, Marburg, unter der Handelsbezeichnung Human-Fibrinogen vertriebene Produkt.

### Gewinnung eines kombinierten Präparates Thrombin und prothrombin:

Prothrombin wird entweder durch Säulenchromatographie aus käuflichem Prothrombinkomplex abgetrennt oder vom Plasma durch Bariumsulfat extrahiert und aus dem kristallinen Niederschlag rückgewonnen.

Daneben sind Thrombin und Prothrombin handelsüblich zugänglich. Beispielsweise kann Thrombin in mikrokristalliner Form mit einer biologischen Aktivität von wenigstens 3 000 Einheiten unter der Handelsbezeichnung "Topostasin" (von Hoffmann La Roche, Grenzach/Baden) bezogen werden. In analoger Weise ist prothrombin als PPSB-präparat von der Firma Immuno AG, Wien erhältlich. Jedes dieser handelüblichen Präparate kann zur Bereitstellung des erfindungsgemäßen, angereicherten Plasmaderivates verwendet werden.

Geeignete, pulverförmige Fibrinolyse-Inhibitoren sind ebenfalls handelsüblich zugänglich. Beispielsweise kann

Aprotinin von der Firma Behring-Werke, Marburg, oder

Trasylol von der Firma Bayer AG, Leverkusen, oder ε-Aminocapronsäure

im Feinchemikalien-Handel bezogen werden. Weiterhin kann ein als Fibrinolyse-Inhibitor geeignetes Antiplasmin, nämlich $\alpha_1$-Antiplasmin, nach nachstehendem Verfahren erhalten werden.

### Gewinnung von $\alpha_1$-Antiplasmin (Fibrinolyse-Inhibitor):

Fibrinogen wird kovalent an Sepharose gebunden und durch Thrombin zu Fibrin umgewandelt. Das so immobilisierte Fibrin dient als Rezeptor für das plasmatische Antiplasmin, das bei Durchlauf von Plasma durch die Säule gebunden wird, und mit Epsilon-Amino-Capronsäure ausgewaschen werden kann.

Auch die gegebenenfalls als weitere Komponenten (Wahlkomponenten) des erfindungsgemäßen, angereicherten Plasmaderivates vorgesehenen Bestandteile sind handelsüblich zugänglich, wie etwa festes, kristallines Albumin (beispielsweise Behring-Werke, Marburg), pulverförmige Prostaglandine (beispielsweise Firma Sigma-Chemie GmbH, München) sowie mikrokristalline, biologisch aktive Gerinnungsfaktoren (beispielsweise Immuno AG, Wien).

Ein wirksames Phospholipid kann beispielsweise entsprechend der nachstehenden Vorschrift erhalten werden.

### Gewinnung eines Phospholipides (Plättchenextrakt):

"Buffy coat" eines Sedimentes aus humanem Vollblut wird mit Seiler-Lösung (Glucose-Salzgemisch) zur Abtrennung der Erythrozyten erschöpfend gewaschen. Das so vorbereitete Leukozyten-Monozyten-Thrombozyten-Präparat wird durch Zusatz von Triton X gelöst, der unlösliche Anteil abzentrifugiert, und die überstehende Lösung mit gesättigtem Ammoniumsulfat bei einem pH-Wert von 7,4 fraktioniert gefällt. Das Sediment wird abzentrifugiert, dialysiert und getrocknet. Der Phospholipidgehalt der Fraktion beträgt etwa 16

bis 25 %. Bei der Prüfung im Thromboplasmintest erweist sich das präparat als gerinnungsaktiv. Die Wachstumssteigerung wird durch Fibroplastenvermehrung in der Kultur überprüft.

Ein anderes brauchbares Phospholipid kann aus Hirn durch Extraktion mit Äther/Chloroform gewonnen werden.

Antibiotika und dgl. sind handelsübliche Präparate.

Alle oben genannten Präparate sind bei Raumtemperatur und bei Temperaturen bis zu 56°C fest und im wesentlichen mikrokristallin. Das erfindungsgemäße, angereicherte Plasmaderivat wird aus diesen Komponenten durch einfaches, trockenes Vermischen erhalten. Das Vermischen kann beispielsweise durch 10 min lange Behandlung in einer Kugelmühle erfolgen. Alternativ kann zum Vermischen eine Ultrabeschallung und Siebung eingesetzt werden. In jedem Falle erhält man ein trockenes, frei fließendes Pulver aus dem homogenen Gemisch der Bestandteile. Nachstehend sind beispielhafte Zusammensetzungen für erfindungsgemäße Präparate angegeben.

### Beispiel 1:

Angereichertes Plasmaderivat zur Unterstützung von Wundverschluß und Wundheilung, bestehend aus

90 g Human-Fibrinogen (erhalten gemäß o.a. Arbeitsweise),

0,5 g Thrombin (bezogen von Behring-Werke, Marburg, biologische Aktivität wenigstens 3000 int. Einheiten pro mg)

0,5 g Trasylol (als Fibrinolyse-Inhibitor, bezogen von der Fa. Bayer AG, Leverkusen);

0,7 g Phospholipid (erhalten gemäß o.a. Arbeitsweise);

8,3 m Albumin (als Trockenhalte- und Stabilisierungsmittel, bezogen von Behring-Werke, Marburg).

Die genannten festen, Pulverförmiges Materialien wurden in eine Kugelmühle gegeben und 10 min lang vermahlen. Man erhält ein homogenes, frei fließendes Pulver, das nach einer Sterilisationsbehandlung, beispielsweise mit Röntgenstrahlung oder Gammastrahlung einer Dosis von 3 kW als angereichertes Plasmaderivat zur akzelerierten Haemostase und zur Unterstützung von Wundverschluß und Wundheilung unmittelbar auf die zu behandelnde Wunde bzw. im Operationsgebiet aufgebracht werden kann.

### Prüfung der Thrombinaktivität des angereicherten plasmaderivates:

Das trockene Pulvergemisch gemäß Beispiel 1 wurde in einer Konzentration von 0,5 mg Pulvergemisch pro 1 ml 0,9-%iger, wässriger

NaCl-Lösung gelöst. 100 µl proben dieser Lösung wurden an einer Standardlösung eines chromogenen Substrates (S2222 der Firma Kabivitrum, Stockholm) getestet. Bei der Endpunktsanalyse muß der Extinktionszuwachs bei 405 nm einer Thrombinaktivität von wenigstens 0,001 internationalen Einheiten entsprechen. Das System wird mit bekannten Thrombinmengen geeicht, so daß es leicht möglich ist, dazwischenliegende Werte zu bestimmen.

Im vorliegenden Falle konnten 0,0025 bis 0,003 Einheiten nachgewiesen werden. Die "Einheiten" haben die Bedeutung, daß 1 Einheit 1 ml einer standardisierten Fibrinogenlösung in 15 sec zum Gerinnen bringen muß.

### Prüfung der Fibrin-Vernetzbarkeit:

Die durch Thrombin gebildeten Fibrin-Gerinnsel wurden sofort erschöpfend in 0,9 %-iger wässriger NaCl-Lösung gewaschen und daraufhin in 0,1 %-iger Monochloressigsäure gelöst. Als Bezugsgröße dient der Extinktionswert bei 280 nm. Die in zeitlich definierten Abständen später aus dem Ansatz entfernten Gerinnsel sind weniger löslich. Ihre Extinktionswerte werden mit dem Nullwert verglichen. Nach 30 min ist das gebildete Fibrin bei 37°C in dem angegebenen Lösungsmittel nicht mehr nachweisbar.

### Prüfung der Gerinnungsaktivität des angereicherten Plasmaderivates:

10 mg Portionen des trockenen Pulvergemisches gemäß Beispiel 1 wurden in einer 5 mM CaCl$_2$-enthaltenden 0,9 %-igen wässrigen NaCl-Lösung unter Rühren gelöst. Die Gerinnungsaktivität dieser Lösung wurde durch die Geschwindigkeit der Fibrinbildung bestimmt. Dazu wurden in definierten Zeitabständen Proben entnommen und in der Elektrophorese auf die Anteile auf Fibrinogen und Fibrinoligomere untersucht. Die Gerinnungszeit beträgt unter den gewählten Bedingungen 70 bis 90 sec. Dabei werden ca. 35 % des Fibrinogens zu Fibrinmonomeren umgewandelt. Die Vernetzung der Fibrinfäden durch den im Präparat enthaltenen Faktor XIII ist innerhalb von 30 min beendet. Danach läßt sich das Präparat in 0,1 %-iger Monochloressigsäure nicht mehr auflösen.

### Beispiel 2:

Die Herstellung des pulverförmigen, angereicherten Plasmaderivates erfolgte im wesentlichen analog zu Beispiel 1. Zusätzlich wurden den dort angegebenen Komponenten in dort angegebenen Anteilen noch 0,5 g Phospholipid aus Hirnsubstanz und 5000 Einheiten Penicillin zugesetzt. Das Vermischen erfolgte analog zu Beispiel 1.

10 mg Portionen des trockenen Pulvergemisches wurden in einer 5 mM CaCl$_2$ enthaltenden 0,9 %-igen NaCl-Lösung unter schnellem Rühren gelöst. Die in der Suspension beginnende Fibrinbildung läßt sich über Trübungsmessungen verfolgen. Die Gelelektrophorese dient zum Nachweis der Fibrinoligomere. Da die Auflösung des Pulvers mit der Fibrinbildung koinzidiert, kann die Gelierung des Ansatzes in der Beobachtungszeit als Maß für die Fibrinbildung herangezogen werden.

Der Zusatz von Phospholipid aus Hirnsubstanz beschleunigt die Gerinnung im anströmenden Blut. Der Ansatz ist in 80 bis 90 sec gelöst.

### Beispiel 3:

Die Herstellung des Pulvergemisches erfolgte im wesentlichen analog zu Beispiel 1. Abweichend wurden 65 g Human-Fibrinogen, 14,0 g Albumin und 18,7 g Globulinfraktion (bezogen von Behring-Werke, Marburg) mit den restlichen dort angegebenen Komponenten in den dort angegebenen Anteilen vermischt.

10 mg Portionen des erhaltenen trockenen Pulvergemisches wurden in CaCl$_2$-enthaltender 0,9%-iger NaCl-Lösung gelöst. Die zu beobachtende Fibrinbildung ist leicht verzögert (Gerinnungszeit 90 bis 120 sec), das gebildete Gel ist plastischer. Der Fibrinkuchen ist aufgrund seiner Elastizität vor allem in Wundbereichen mit hoher mechanischer Beanspruchung durch Muskelkraft und -kontraktion einsetzbar, so zum Beispiel bei tiefen Hautwunden an den Extremitäten oder bei Sehnenrupturen, die nach Auskämmen der Kollagenfasern mittels dem sich verflüssigenden Pulver durch die Fibrinpolymerisate zusammengehalten werden können.

### Beispiel 4:

Die Herstellung des Pulvergemisches erfolgte im wesentlichen analog zu Beispiel 1. Abweichend wurden 96 g Human-Fibrinogen und 2,29 g Albumin mit den restlichen dort angegebenen Komponenten in den dort angegebenen Anteilen vermischt. Das aus diesem Plasmaderivat gebildete Fibringerinnsel erweist sich als steifer, weniger elastisch und außerordentlich druckstabil. Der Wundverschluß wird durch die hohe Fibrinkonzentration beschleunigt (Gerinnungszeit 50 bis 70 sec), so daß dieses Wundverschlußpuder bei stärker blutenden Traumen, Gefäßrupturen oder Hautverletzungen eingesetzt werden kann, die durch eine hohe Ausströmgeschwindigkeit des Kapillarblutes charakterisiert sind.

**Beispiel 5:**

Angereichertes Plasmaderivat zur Unterstützung von Wundverschluß und Wundheilung, bestehend aus

85 Gew.-% Fibrinogen (handelsübliches Präparat mit weniger als 2 Gew.-% kälteunlösliches Globulin, bezogen von Behring-Werke, Marburg);

4 Gew.-% Thrombin ("Topostasin" von Hoffmann La Roche, Granzach/Baden; Aktivität: wenigstens 3000 int.Einheiten pro mg);

5 Gew.-% Prothrombin (PPSB-Präparat, bezogen von Immuno AG, Wien); 1 Gew.-% Fibrinolyse-Inhibitor, nämlich ein 1:1-Ge-misch aus $\alpha_1$-Antiplasmin (hergestellt wie oben angegeben, und $\alpha_2$-Makroglobulin (bezogen von Behring-Werke, Marburg);

2 Gew.-% Phospholipid aus Hirnsubstanz. Zur Gewinnung wurde Hirngewebe vom Rind oder Schwein von den Hirnhäuten befreit, blutfrei gewaschen, lyophilisiert und gepulvert; das Pulver wurde mit Chloroform/Äther extrahiert, der Extrakt eingedampft und der dabei erhaltene Rückstand zerkleinert und verwendet;

3 Gew.-% Globulinfraktion ($\alpha$-, $\beta$-, $\gamma$-Globulin-Gemisch, bezogen von Böhringer, Mannheim).

Die angereicherten Plasmaderivate gemäß den Beispielen 2,3,4 und 5 ergeben hinsichtlich der Thrombinaktivität, der Fibrinvernetzbarkeit und der Gerinnungsaktivität ähnliche Ergebnisse, wie sie oben für Beispiel 1 angegeben sind.

Wie bereits oben ausgeführt, kann das angereicherte Plasmaderivat in Form eines trockenen Pulvergemisches nach üblicher Sterilisationsbehandlung unmittelbar auf den zur Verklebung vorgesehenen Gewebeteilen oder der zu stillenden Blutung aufgebracht werden. Das Pulver kann hierzu auf die feuchte Applikationsstelle aufgestreut werden. Weiterhin kann das pulverförmige, angereicherte Plasmaderivat in einem Schnellverband integriert werden.

Als Verbandmaterial kommen natürliche oder synthetische Verbandmaterialien in Betracht, wobei jedoch Kollagen als Trägermaterial ausdrücklich ausgenommen ist. Beispielsweise kann das trockene Pulvergemisch mittels einem sterilen Gasstrahl auf Verbandmull aufgeblasen werden. Das staubfeine Pulver haftet in ausreichender Menge an der großen Oberfläche des Verbandmaterials. Vorzugsweise werden etwa 0,5 mg angereichertes Plasmaderivat pro $cm^2$ Oberfläche Trägermaterial aufgebracht. Ein derartig präpariertes Fibrinvlies kann direkt zur Wundbehandlung verwendet werden, oder es kann in einem Schnellverband (Heftpflaster) integriert werden. Die Anwendung natürlicher oder synthetischer Verbandsmaterialien vergrößert die Raktionsfläche und erleichtert das Abdecken größerer Wundgebiete.

Nach einer weiteren, alternativen Applikationsmethode kann das angereicherte Plasmaderivat mittels eines, das Plasmaderivat nicht lösenden Treibmittels versprüht werden.

Hierzu kann beispielsweise ein Spray vorgesehen werden, der 100 mg, im Vakuum getrocknetes, angereichertes Plasmaderivat, beispielsweise das Pulvergemisch nach obigem Beispiel 1, in 10 ml Suspensionsmittel enthält. Als Suspensionsmittel kann beispielsweise ein Äthanol/Äther-Gemisch (8 Vol.-Teile Äthanol auf 2 Vol.-Teile Äther) oder Frigen 114 dienen. Die erhaltene Suspension wird in eine Verteilerdose mit aufgeschraubtem Kolbenzungen-Sprühverteiler abgefüllt.

Eine weitere Applikationsform besteht im Auftrag eines, das angereicherte Plasmaderivat enthaltenden Schaumes. Zur Bereitung eines solchen Schaumes können 100 mg angereichertes Plasmaderivat, beispielsweise das Pulvergemisch nach obigem Beispiel 3, in 10 ml Träger, etwa einem Gemisch aus 1 Vol.-Teil Phospholipid und 9 Vol.-Teile Glyzerin suspendiert, und diese Suspension mittels Kohlendioxid, das üblichen $CO_2$-Gaspatronen entnommen wird, verschäumt werden.

Das Verstäuben des pulverförmigen Plasmaderivates, das Versprühen in Form eines Sprays oder das Verschäumen eines entsprechenden Schaumes erlauben das Einbringen und Abdichten von schwierig zugänglichen Gewebespalten und/oder Cavitäten.

Das erfindungsgemäße, angereicherte Plasmaderivat ist dank seines festen Zustandes bei Abwesenheit von Feuchtigkeit hervorragend lagerfähig und kann unter trockenen, sterilen Bedingungen wenigstens 2 Jahre gelagert werden, ohne mehr als 10 % der biologischen Aktivität zu verlieren.

Die biologische Aktivität bei der Gewebeverklebung sowie die Umsetzung zu einer, die Blutung stillenden Fibrin-Wundauflage setzt ein, nachdem das trockene, pulverformige Plasmaderivat in Körperflüssigkeit an- und aufgelöst ist. Bereits nach kurzer Zeit, beispielsweise nach 2 min, tritt eine akzelerierte Haemostase ein. Die biochemische Regelung des Wundverschlusses wird durch das erhöhte Angebot an Fibrinogen, Fibrinolyseinhibitoren verstärkt und optimiert. Die Zugabe von Plättchenfaktoren stimuliert die Gerinnung des ausströmenden Blutes, die enthaltenen Wachstumsfaktoren optimieren die Wundheilung.

Jede blutende Wunde liefert gerinnbares Material, das wegen der Strömungsgeschwindigkeit von den Wundrändern weggespült wird. Gerinnbare, trockene Wundpuder erhöhen lokal das Gerinnungspotential, saugen Flüssigkeit auf und fördern die Plättchenadhäsion. Das im Wundgebiet exponierte Kollagen adsorbiert das Fibringerinnsel und verstärkt die Haftung des Wundverschlußmaterials. Durch die trockene Applikationsform erübrigt sich eine besondere Aufbewahrung oder eine Vermischung mit Thrombin. Das Verkleben von Hautlappen, Sicherung von Operationsnähten oder das Unterbinden von Sickerblutungen wird durch die Sprayform besonders einfach in der Handhabung.

## Patentansprüche

1. Angereichertes Plasmaderivat in Form eines zur Unterstützung von Wundverschluß und Wundheilung bestimmten biochemischen Substrates, wobei die Anwendung auf Kollagen als Trägermaterial ausgenommen ist, und dessen Zusammensetzung einen Gehalt an Fibrinogen, Thrombin, Komponenten des Prothrombinkomplexes und Proteaseinhibitoren einschließt und im Hinblick auf eine optimierte Aktivierung des exogenen und/oder endogenen Gerinnungssystems und unter Berücksichtigung einer Vielzahl physiologischer und gegebenenfalls pathologischer Gesichtspunkte ausgewählt ist, dadurch gekennzeichnet, daß das Substrat gegebenenfalls zusätzlich Beimischungen von Blutplättchenextrakten, Antibiotika und dergleichen enthalten kann, alle Substratbestandteile ausnahmslos in pulverförmigem Zustand vorliegen, und das Substrat zur Applizierung auf einer Wunde bzw. im Operationsgebiet als trockenes Pulvergemisch oder zusammen mit einem die Substratbestandteile nicht lösenden Treibmittel als Spray oder Schaum konfektioniert ist.

2. Plasmaderivat nach Anspruch 1, wobei
a) der Fibrinogengehalt 60 bis 96 Gew.-% ausmacht;
b) das Fibrinogen weitgehend frei von kälteunlöslichem Globulin ist;
c) der Gehalt an Thrombin und/oder Prothrombin 0,1 bis 15 Gew.-% ausmacht;
d) der Gehalt an Fibrinolyse-Inhibitor 0,5 bis 5 Gew.-% ausmacht;
e) gegebenenfalls zusätzlich Phospholipide, Prostaglandine, Trockenhalte- und Stabilisierungsmittel, Antibiotika und/oder Blutgerinnungsfaktoren vorhanden sind;
f) alle Komponenten in biologisch aktiver, bei Temperaturen bis zu 56°C fester, pulverförmiger Form vorliegen; und
g) diese festen Komponenten miteinander vermischt sind.

3. Plasmaderivat nach Anspruch 2, wobei das Fibrinogen aus Humanplasma durch Fällung mit einem Glycin, β-Alanin und Äthanol enthaltendem Lösungsmittelgemisch und nachfolgender Dialyse und Lyophilisation des Fällungsproduktes erhalten worden ist.

4. Plasmaderivat nach Anspruch 2 oder 3, wobei das Fibrinogen weniger als 2 Gew.-% kälteunlösliches Globulin enthält.

5. Plasmaderivat nach einem der Ansprüche 2 bis 4, wobei der Fibrinolyse-Inhibitor ein Antiplasmin ist.

6. Plasmaderivat nach einem der Ansprüche 2 bis 5, wobei das Prothrombin zu wenigstens 95 % in Thrombin umwandelbar ist.

7. Plasmaderivat nach einem der Ansprüche 2 bis 6, wobei das Thrombin eine biologische Aktivität von wenigstens 1000 internationalen Einheiten pro mg aufweist.

8. Plasmaderivat nach einem der Ansprüche 2 bis 7, wobei auf ein Gew.-Teil Thrombin 0,1 bis 2 Gew.-Teile Prothrombin kommen.

9. Plasmaderivat nach einem der Ansprüche 2 bis B, wobei das Plasmaderivat 80 bis 94 Gew.-% Fibrinogen, 1 bis 10 Gew.-% Thrombin und/oder Prothrombin, und 0,01 bis 3 Gew.-% Fibrinolyse-Inhibitor, sowie weniger als 0,4 Gew.-% kälteunlösliches Globulin enthält.

10. Plasmaderivat nach einem der Ansprüche 1 bis 9, wobei das Plasmaderivat als Spray oder Schaum konfektioniert ist.

11. Verfahren zur Herstellung eines angereicherten Plasmaderivates, in Form eines zur Unterstützung von Wundverschluß und Wundheilung bestimmten biochemischen Substrates, wobei die Anwendung auf Kollagen als Trägermaterial ausgenommen ist, dessen Zusammensetzung einen Gehalt an Fibrinogen, Thrombin, Komponenten des Prothrombinkomplexes und Prothease-Inhibitoren einschließt, und im Hinblick auf eine optimierte Aktivierung des exogenen und/oder endogenen Gerinnungssystems und unter Berücksichtigung einer Vielzahl physiologischer und gegebenenfalls pathologischer Gesichtspunkts ausgewählt ist, dadurch gekennzeichnet, daß die in biologisch aktiver, bei Temperaturen bis zu 56°C in fester, pulverförmiger Form vorliegenden Komponenten, nämlich 60 bis 96 Gew.-Teile von kälteunlöslichem Globulin weitgehend freies Fibrinogen, 0,1 bis 15 Gew.-Teile Thrombin und/oder Prothrombin, 0,5 bis 5 Gew.-Teile Fibrinolyse-Inhibitor und gegebenenfalls Zusätze von Phospholipiden, Prostaglandinen, Trockenhalte- und Stabilisierungsmittel, Antibiotika und/oder Blutgerinnungsfaktoren trocken miteinander vermischt und zur Applizierung in trockenem Zustand auf einer Wunde oder im Operationsgebiet konfektioniert werden.

12. Verfahren nach Anspruch 11, wobei
80 bis 94 Gew.-Teile Fibrinogen,
1 bis 10 Gew.-Teile Thrombin und/oder Prothrombin,
0,01 bis 3 Gew.-Teile Fibrinolyse-Inhibitor und gegebenenfalls Zusätze von Phospholipiden, Prostaglandinen, Trockenhalte- und Stabilisierungsmittel, Antibiotika und/oder Blutgerinnungsfaktoren trocken miteinander vermischt werden.

13. Verfahren nach Anspruch 11 oder 12, wobei die Komponenten und die gegebenenfalls vorhandenen Zusätze zum Vermischen 10 min lang in einer Kugelmühle behandelt werden.

14. Verfahren nach Anspruch 11 oder 12, wobei die Komponenten und die gegebenenfalls vorhandenen Zusätze zum Vermischen mit Ultraschall behandelt und gesiebt werden.

15. Verfahren nach Anspruch 11 oder 12, wobei das trockene pulverförmige Gemisch zum Zerstäuben, Versprühen oder Verschäumen mittels eines Treibmittels konfektioniert wird.

## Claims

1) An enriched plasma derivative in form of a biochemical substrate for supporting wound closure and wound healing, wherein the application on collagen as carrier material is excluded, and

the composition thereof includes fibrinogen, thrombin, components of the prothrombin complex and protease inhibitors and is selected with regard to an optimized activation of the exogenic and/or endogenic coagulation system as well as under consideration of a number of physiological and - if applicable - also pathological aspects,

characterized in

that said substrate optionally may contain in addition admixtures of blood platelet extracts, antibiotics and the like;

that - without any exception - all substrate constituents being present in a powdery form; and for application on a wound or in the area of operation, said substrate being in the form of a dry powdery mixture or - in combination with a propellant which will not dissolve the substrate constituents - being in the form of a spray or foam.

2) The plasma derivative according to claim 1, wherein

a) the content of fibrinogen amounts of from 60 to 90 % by weight;

b) the fibrinogen is largely free from cryo-insoluble globulin;

c) the content of thrombin and/or prothrombin amounts of from 0.1 to 15 % by weight;

d) the content of fibrinolysis inhibitor amounts of from 0.5 to 5 % by weight;

e) optionally in addition thereto a phospholipid, a prostaglandin, a desiccating and stabilizing agent, an antibiotic and/or coagulation factor being present;

f) all components being present in biologically active, solid powdery form at temperatures up to 56° C; and

g) these solid components are mixed with each other.

3) The plasma derivative according to claim 2, wherein the fibrinogen is a product obtained from human plasma by precipitation with a mixed solvent containing glycine β-alanine and ethanol and subsequent dialysis and lyophilization of the precipitate.

4) The plasma derivative according to claim 2 or 3, wherein the fibrinogen contains less than 2 % by weight of cryo-insoluble globulin.

5) The plasma derivative according to anyone of the claims 2 to 4,

wherein the fibrinolysis inhibitor is an antiplasmin.

6) The plasma derivative according to anyone of the claims 2 to 5,

wherein prothrombin being present and convertible into thrombin to at least 95 %.

7) The plasma derivative according to anyone of the claims 2 to 6,

wherein thrombin being present and has a biological activity of at least 1,000 international units per mg.

8) The plasma derivative according to anyone of the claims 2 to 7,

wherein both thrombin and prothrombin being present, and 0,1 to 2 parts by weight of prothrombin are provided per 1 part by weight of thrombin.

9) The plasma derivative according to anyone of the claims 2 to 8,

wherein the plasma derivative contains 80 to 94 % by weight fibrinogen; 1 to 10 % by weight thrombin and/or prothrombin; 0.01 to 3 % by weight fibrinolysis inhibitor; and less than 0.4 % by weight cryo-insoluble globulin.

10) The plasma derivative according to anyone of the claims;; 1 to 9

wherein the plasma derivative being in the form of a spray or foam.

11) A method for preparing an enriched plasma derivative in form of a biochemical substrate for supporting wound closure and wound healing wherein the application on collagen as carrier material is excluded, and

the composition thereof includes fibrinogen, thrombin, components of the prothrombin complex and protease inhibitors and is selected with regard to an optimized activation of the exogenic and/or endogenic coagulation system as well as under consideration of a number of physiological and - if applicable - also pathological aspects,

characterized in

that the components, namely

60 to 96 parts by weight fibrinogen which is largely free from cryo-insoluble globulin,

0.1 to 15 parts by weight thrombin and/or prothrombin,

0.5 to 5 parts by weight fibrinolysis inhibitor, and optionally additions of a phospholipid a prostaglandin a desiccating and stabilizing agent an antibiotic and/or coagulation factor, being present in biologically active, solid, powdery form at temperatures up to 56° C, and are mixed together in dry form and are prepared to be applied on a wound or in the area of operation in a dry form.

12) The method according to claim 11, wherein 80 to 94 parts by weight fibrinogen, 1 to 10 parts by weight thrombin and/or prothrombin 0.01 to 3 parts by weight fibrinolysis inhibitor and optionally additions of a phospholipid, a prostaglandin a desiccating and stabilizing agent, an antibiotic and/or coagulation factor are mixed together in a dry form.

13) The method according to claim 11 or 12, wherein the components and the optionally provided additions are mixed by treating up to 10 minutes in a ball mill.

14) The method according to claim 11 or 12, wherein the components and the optionally provided additions are mixed by ultrasonic treatment and are sieved.

15) The method according to claim 11 or 12, wherein the dry powdery mixture is prepared with a propellant in order to be atomized sprayed or foamed.

## Revendications

1. Dérivé enrichi du plasma sous forme d'un substrat biochimique destiné à favoriser la fermeture de plaies et à les guérir, l'utilisation de collagène comme matière support étant exclue, et dont la composition renferme une teneur en fibrinogène, en thrombine, en constituants du complexe de prothrombine et en inhibiteurs de protéase et est choisie en vue d'une activation optimale du système de coagulation exogène et/ou endogène et en tenant compte d'un grand nombre de points de vue physiologiques et le cas échéant pathologiques,
caractérisé en ce que
le substrat peut contenir, le cas échéant, en plus, des additions d'extraits de plaquettes du sang, d'antibiotiques et de produits similaires, tous les constituants du substrat se présentent sans exception sous forme pulvérulente, et
le substrat est conditionné en vue d'une application sur une plaie et dans le domaine opératoire en tant que mélange pulvérulent sec ou avec un agent propulseur ne dissolvant pas les constituants du substrat, sous la forme d'un spray ou d'une mousse.

2. Dérivé du plasma suivant la revendication 1, dans lequel
a) la teneur en fibrinogène représente de 60 à 96 % en poids;
b) le fibrinogène est essentiellement exempt de globuline insoluble à froid;
c) la teneur en thrombine et/ou en prothrombine représente de 0,1 à 15 % en poids;
d) la teneur en inhibiteur de fibrinolyse représente de 0,5 à 5 % en poids;
e) il est prévu, éventuellement, en plus des phospholipides, des prostaglandines, des agents de siccité et de stabilisation, des antibiotiques et/ou des facteurs de coagulation du sang;
f) tous les constituants se présentent sous une forme active biologiquement, pulvérulente, solide à des températures allant jusqu'à 56° C; et
g) ces constituants solides sont mélangés les uns aux autres.

3. Dérivé du plasma suivant la revendication 2, dans lequel le fibrinogène a été obtenu à partir du plasma humain par précipitation par un mélange de solvants contenant de la glycine, de la β - alanine et de l'éthanol et ensuite par dialyse et lyophilisation du produit de précipitation.

4. Dérivé du plasma suivant la revendication 2 ou 3,
dans lequel le fibrinogène contient moins de 2 % en poids de globuline insoluble à froid.

5. Dérivé du plasma suivant l'une des revendications 2 à 4,
dans lequel l'inhibiteur de fibrinolyse est une antiplasmine.

6. Dérivé du plasma suivant l'une des revendications 2 à 5, dans lequel la prothrombine peut être transformée pour au moins 95 % en thrombine.

7. Dérivé du plasma suivant l'une des revendications 2 à 6,
dans lequel la prothrombine a une activité biologique d'au moins 1000 unités internationales par mg.

8. Dérivé du plasma suivant l'une des revendications 2 à 7,
dans lequel la prothrombine représente de 0,1 à 2 parties en poids pour une partie en poids de thrombine.

9. Dérivé du plasma suivant l'une des revendications 2 à 8,
dans lequel le dérivé du plasma contient de 80 à 94 % en poids de fibrinogène, de 1 à 10 % en poids de thrombine et/ou de prothrombine, et
de 0,01 à 3 % en poids d'inhibiteur de fibrinolyse, ainsi que
moins de 0,4 % en poids de globuline insoluble à froid.

10. Dérivé du plasma suivant l'une des revendications 1 à 9,
dans lequel le dérivé du plasma est conditionné sous forme de spray ou de mousse.

11. Procédé de préparation d'un dérivé enrichi du plasma,
sous forme d'un substrat biochimique destiné à favoriser la fermeture de plaies et à les guérir, l'utilisation de collagène comme matière support étant exclue, et dont la composition renferme une teneur en fibrinogène, en thrombine, en constituants du complexe de prothrombine et en inhibiteurs de protéase et est choisie en vue d'une activation optimale du système de coagulation exogène et/ou endogène et en tenant compte d'un grand nombre de points de vue physiologiques et le cas échéant pathologiques, caractérisé en ce que
il consiste à mélanger entre eux à sec, les constituants se présentant sous une forme active biologiquement, pulvérulente, solide jusqu'à des températures allant jusqu'à 56° C, à savoir
de 60 à 96 parties en poids de fibrinogène essentiellement exempt de globuline insoluble à froid,
de 0,1 à 15 parties en poids de thrombine et/ou de prothrombine,
de 0,5 à 5 parties en poids d'inhibiteurs de fibrinolyse et, le cas échéant, des additions de phospholipides, de prostaglandines, d'agents de, siccité et de stabilisation, d'antibiotiques et/ou de facteurs de coagulation du sang, et à les conditionner en vue d'une application à l'état sec sur une plaie ou dans le domaine opératoire.

12. Procédé suivant la revendication 11 qui consiste
à mélanger entre eux à sec
de 80 à 94 parties en poids de fibrinogène, de 1 à 10 parties en poids de thrombine et/ou de prothrombine,
de 0,01 à 3 parties en poids d'inhibiteur de fibrinolyse, et le cas échéant des additions de phospholipides, de drostaglanlines, d'agents de siccite et de stabilisation, d'antibiotiques et/ou de facteurs de coagulation du sang.

13. procédé suivant la revendication 11 ou 12, qui consiste à traiter les constituants et les additifs éventuellement présents pour le mélange pendant 10 minutes dans un broyeur à boulets.

14. Procédé suivant la revendication 11 ou 12, qui consiste à traiter aux ultra-sons et à tamiser les constituants et les additifs éventuellement présents pour le mélange

15. Procédé suivant la revendication 11 ou 12, qui consiste à conditionner le mélange pulvérulent sec en vue de le réduire en poudre, de le pulvériser ou de le mettre sous forme de mousse au moyen d'un agent propulseur.